# EUROPEAN PATENT SPECIFICATION

(11) **EP 4 601 530 B1**
(45) Date of publication and mention of the grant of the patent: **09.12.2026**
(21) Application number: 23786272.7
(22) Date of filing: 11.10.2023
(51) Int. Cl.: A61B 5/00, G06T 7/00, A61B 5/024, A61B 5/08

(54) **DETECTION OF VITAL SIGNS FROM IMAGES**
ERKENNUNG VON VITALZEICHEN AUS BILDERN
DÉTECTION DE SIGNES VITAUX À PARTIR D'IMAGES

(30) Priority: 12.10.2022 EP 22201161
(43) Date of publication of application: 20.08.2025
(73) Proprietor: Koninklijke Philips N.V., 5656 AE Eindhoven (NL)
(72) Inventor: COEZIJN, Etienne René Eveline, 5656 AG Eindhoven (NL); ROCQUE, Mukul Julius, 5656 AG Eindhoven (NL); VAN DALFSEN, Age Jochem, 5656AG Eindhoven (NL)
(74) Representative: Philips Intellectual Property & Standards
(86) International application number: PCT/EP2023/078105
(87) International publication number: WO 2024/079152

(56) References cited:
- US-A1- 2013 321 678
- US-A1- 2014 086 462
- US-A1- 2014 303 454
- US-A1- 2020 178 809

## Description

### FIELD OF THE INVENTION

The invention relates to detection of a vital sign for a person from one or more images, and in particular, but not exclusively, to detection of a pulse and/or respiratory vital signs from images captured of a face, chest, and/or skin area of a person.

### BACKGROUND OF THE INVENTION

Detection of vital signs based on images of a person has become a highly advantageous and useful tool in many scenarios.

Image based vital sign monitoring seeks to measure vital signs from visual cues captured by images of the person. Such vital signs may specifically include pulse or respiratory properties. Application areas include, but are not limited to, emergency units and department triage, (infectious disease) screening applications, and telehealth solutions, etc.

Different approaches and algorithms for extracting vital sign data from images are known in the art. For example, remote photoplethysmography, rPPG, is a technique used to determine a blood volume pulse signal from images of skin tissue, e.g. typically captured by using custom or off-the-shelf cameras, as known from US2014/303454A1.

As another example, respiratory properties, such as respiratory signal and/or respiratory rate may be estimated from images using e.g. detection of changes in the image caused by chest/abdomen movements.

Such vital sign monitoring is highly advantageous in many scenarios as it may allow a practical and non-intrusive monitoring and detection of vital signals.

In order to provide optimal detection of the vital signs, it is important that the images have suitable properties for the specific detection/ estimation. This may for example require capture properties for the camera to be carefully set to ensure an appropriate capture operation. In particular, the adaptation of settings and calibrations of optical parameters and sensor parameters are critical in order to achieve a high quality imaging of the person, and especially to ensure the captured images are suitable for detection of vital signs. In particular, adapting camera properties and settings prior to digitization of sensor signals may be essential in ensuring that the captured images allow accurate and reliable determination of vital signs. Such considerations may include adapting the camera properties and settings to prevent clipping, to exploit the dynamic range effectively, to achieve improved signal to noise ratio, to adapt relationships between different color channels, to allow efficient detection of visual properties suitable for estimation of vital signs etc.

The adaptation of the settings and parameters may be particularly challenging to optimize for the detection of vital signs as this may differ from optimal settings and parameters that seek to provide images which are visually as realistic as possible and which are optimized for perception by the human visual system. The optimum settings for detecting vital signs may be different. For example, detecting the pulse of a person may be more reliable if the detection is based on images that exaggerate the visual properties that change in line with the pulse.

Hence, an improved approach for determining vital signs from captured images would be advantageous. In particular, an approach that allows improved operation, increased flexibility, improved determination of vital signs, improved representation of visual properties allowing vital signs to be determined, reduced sensitivity to variations and light conditions in the capturing environment, reduced complexity, facilitated implementation, reduced memory and/or storage requirements, and/or improved performance and/or operation would be advantageous.

### SUMMARY OF THE INVENTION

Accordingly, the Invention seeks to preferably mitigate, alleviate or eliminate one or more of the above mentioned disadvantages singly or in any combination.

There is provided apparatuses and methods as defined in the appended claims.

According to an aspect of the invention there is provided an apparatus for estimating vital signs of a person from video images, the apparatus comprising: a receiver arranged to receive video images comprising a plurality of color channels, each color channel representing a digitization of an optical sensor signal from a color channel optical sensor; a first detector arranged to determine a first image area in at least one image of the video images, the first image area corresponding to a skin area of the person; a second detector arranged to determine a second image area in at least one image of the video images, the second image area corresponding to a different area of the person than the first image area; a determiner arranged to determine a set of color channel distributions for pixels of the first image area; a processor arranged to provide a skin tone indication, the skin tone indication being indicative of a skin tone of the person; a reference processor arranged to provide a reference color channel distribution property for the skin tone; a gain processor arranged to determine gains for the optical sensor signals in dependence on a luminance property determined for the second image area and in dependence on a difference between the reference color channel distribution property and a property of the set of color channel distributions, wherein the gains are determined so as to reduce the difference; a gain controller arranged to control gain values applied to the optical sensor signals to match the determined gains; and a vital sign determiner arranged to estimate a vital sign property for the person from the video images.

The invention may in many embodiments allow improved determination of vital signs based on images. The approach may allow improved adaptation to the specific environmental conditions, and in particular to light conditions. The approach may in particular allow an improved adaptation for the specific purpose of detecting vital signs rather than e.g., just optimizing realism of captured video images. The approach may in many scenarios facilitate operation and may e.g., allow improved user convenience as requirements for manual adaptations and/or accurate control of the capture environment can be reduced. In many scenarios, the approach may allow a capture operation to particular focus on visual properties suitable for determining vital signs thereby typically improving the reliability and accuracy of the detection.

The approach may provide improved camera/ capture control in many embodiments. The sensitivity of a capture operation to variations in light conditions and the variety of people for which vital signs are determined may often be reduced substantially.

The color channels may specifically be Red, Green, and Blue color channels, and the video images may be RGB images.

The first image area and the second image area may typically be in the same image of the video images but may in some embodiments and scenarios be in different images. A video image may be a frame of a video signal.

The property of the set of color channel distributions may comprise a set of values with each value being for one color channel of the plurality of color channels. The reference color channel distribution property may comprise a set of values with each value being for one color channel of the plurality of color channels.

The vital sign may in many embodiments be a pulse rate and/or a respiratory rate. The second image area may be an image area corresponding to a chest area of the person.

The property of the set of color channel distributions may be a set of one or more (pixel) percentile values for a color channel, and specifically may be a set of one or more pixel values corresponding to a given percentile of the pixel values of the color channel.

The apparatus may be a camera apparatus.

According to an optional feature of the invention, the apparatus may further comprise: a gain circuit arranged to apply the gain values to optical sensor signals from at least two color channel optical sensors; and an image generator coupled to the gain circuit and arranged to generate the video images from the optical sensor signals received from the gain circuit.

The approach may provide improved operation and/or facilitated implementation in many embodiments, and may in particular provide improved capturing of images for vital sign determination.

According to an optional feature of the invention, the reference color channel distribution property comprises a set of reference intensity values for different color channels, and the property of the color channel distributions comprises a set of determined intensity values for the set of color channel distributions.

This may provide improved operation and/or facilitated implementation in many embodiments. In particular, it may typically provide an efficient adaptation of a capture operation for determining vital signs.

The determined intensity values may specifically be determined pixel values, and may specifically be intensity values/ pixel values for a given percentile of the distribution.

According to an optional feature of the invention, the determined intensity values are color channel pixel values for a given percentile of the set of color channel distributions.

This may provide improved operation and/or facilitated implementation in many embodiments.

According to an optional feature of the invention, the gain processor arranged to determine a gain for a first color channel of the plurality of color channels in dependence on a difference between a determined intensity value for the first color channel and a reference intensity value for the first color channel value.

This may provide improved operation and/or facilitated implementation in many embodiments. In particular, it may typically provide an efficient adaptation of a capture operation for determining vital signs.

According to an optional feature of the invention, gain processor arranged to set the gains such that a relationship between determined intensity values for the different color channels match a relationship between reference intensity values for the different color channels.

This may typically allow a particularly efficient color channel balance (e.g. white balance) that can be optimized for the specific algorithm used to determine the vital signs.

According to an optional feature of the invention, the luminance property is a luminance for the second image area resulting from applying the determined gains to the second image area, and the gain processor is arranged to set the gains subject to the luminance property being indicative of a luminance for the second image area meeting a criterion.

This may provide efficient adaptation. The luminance criterion may specifically include a requirement that the luminance is within a given luminance range.

According to an optional feature of the invention, the gain processor is arranged to determine a common gain component in dependence on the luminance property and relative gain components in dependence on the comparison of the reference color channel distribution property and a property of the set of color channel distributions, the common gain component being a gain applied to all color channels of the plurality of color channels and the relative gain components being gain components applied to individual color channels of the plurality of color channels.

This may provide improved operation and/or facilitated implementation in many embodiments. It may allow not only an efficient adaptation of the capture operation but also a practical yet flexible implementation.

According to an optional feature of the invention, the gain processor is arranged to control an exposure property of at least one color channel optical sensor in dependence on at least one of the luminance property and the comparison of the reference color channel distribution property and a property of the set of color channel distributions.

This may typically allow an improved adaptation and may for example allow adaptation over a more diverse set of capture scenarios and light conditions. It may often improve image quality and allow e.g. higher signal to noise ratio for the optical sensor signals and/or captured images.

This may provide improved operation and/or facilitated implementation in many embodiments.

According to an optional feature of the invention, the gain processor is arranged to determine and store a first color channel distribution for a third image area of an image for which a skin area has been detected; and to determine gains for the optical sensor signals for a first image for which no skin area has been detected in dependence on the first color channel distribution and a second color channel distribution being determined for a third image area for the first image.

This may provide improved operation in many embodiments and scenarios. It may typically allow suitable adaptation to be performed even for images/ times for which no skin area can be detected in the video images. It may often ensure that the capture operation is adapted to provide improved conditions for detecting image areas corresponding to skin areas. For example, it may ensure that the capture operation for times when no skin is detected (e.g. by a face detector) is such that the captured images are suitable for performing a skin detection operation (e.g. specifically a face detection).

According to an optional feature of the invention, the gain processor is arranged to set the gain to reduce a difference between a property of the first color channel distribution and the second color channel distribution.

This may provide improved operation and/or facilitated implementation in many embodiments.

According to an optional feature of the invention, the third image area is a full image area.

According to an optional feature of the invention, the gain processor is arranged to determine initial gains for the optical sensor signals in dependence on comparison of a full image intensity value to a predetermined reference intensity value; the full image intensity value being an intensity value for a predetermined percentile of a color channel of the plurality of color channels.

This may allow improved initial adaptation and operation, e.g. such that images can be captured that are suitable for initial skin area detection etc.

According to an aspect of the invention there is provided a method of estimating vital signs of a person from images, the method comprising: receiving video images comprising a plurality of color channels, each color channel representing a digitization of an optical sensor signal from a color channel optical sensor; determining a first image area in at least one image of the video images, the first image area corresponding to a skin area of the person; determining a second image area in at least one image of the video images, the second image area corresponding to a different area of the person than the first image area; determining a set of color channel distributions for pixels of the first image area; providing a skin tone indication, the skin tone indication being indicative of a skin tone of the person; providing a reference color channel distribution property for the skin tone; determining gains for the optical sensor signals in dependence on a luminance property determined for the second image area and in dependence on a difference between the reference color channel distribution property and a property of the set of color channel distributions, wherein the determined gains are determined so as to reduce the difference; controlling gain values applied to the optical sensor signals to match the determined gains; and estimating a vital sign property for the person from the video images.

These and other aspects, features and advantages of the invention will be apparent from and elucidated with reference to the embodiment(s) described hereinafter.

### BRIEF DESCRIPTION OF THE DRAWINGS

Embodiments of the invention will be described, by way of example only, with reference to the drawings, in which
FIG. 1 illustrates an example of elements of a camera in accordance with some embodiments of the invention;
FIG. 2 illustrates an example of elements of a camera in accordance with some embodiments of the invention;
FIG. 3 illustrates an example of elements of a camera in accordance with some embodiments of the invention;
FIG. 4 illustrates some elements of a possible arrangement of a processor for implementing elements of audio apparatus in accordance with some embodiments of the invention.

### DETAILED DESCRIPTION OF THE EMBODIMENTS

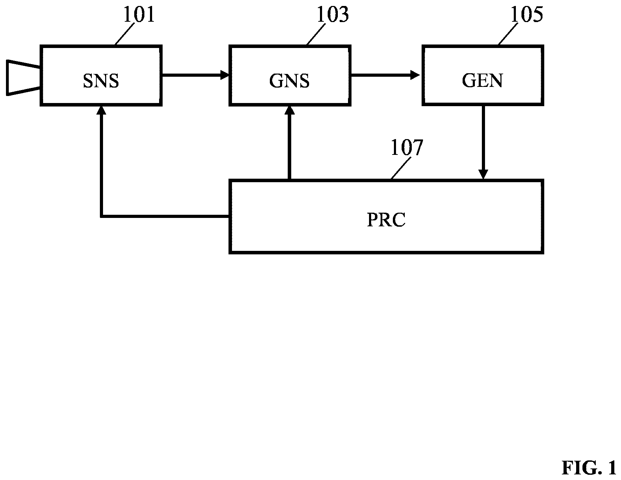

Fig. 1 illustrates an example of a camera apparatus arranged to capture images of a person and to determine vital signs for a person from the captured images. Vital signs that may be detected by the apparatus may include pulse rate, and respiratory rate.

The vital signs are detected from a video sequence of images/ frames and may specifically be detected based on variations that occur in the images. Thus, an optical camera can be positioned to capture a person/ patient and the resulting video images may be analyzed and compared to each other to detect the vital sign. For example, photoplethysmography may be used to determine volumetric variations of blood circulation.

In order to achieve an accurate and reliable detection of vital signs, it is critical that the capture of the images is suitably adapted to provide images that have suitable optical properties for detecting visible cues that can allow the vital sign to be detected. Such adaptation of the capture operation may be critical to the accuracy and reliability that can be achieved for the determination of vital signs.

In the example of FIG. 1, the camera apparatus comprises a plurality of optical sensors 101 that generate optical sensor signals for different color channels. At least two optical sensors are thus arranged to capture visible light in different bandwidths/ with different frequency sensitivities. In many embodiments, the camera apparatus comprises three optical sensors capturing spectra corresponding to respectively Red, Green, Blue (RGB) color channels. The optical sensors 101 accordingly generates optical sensor signal corresponding to an RGB color signal with RGB color channels.

The sensors are coupled to a gain circuit 103 which is arranged to apply gains to the optical sensor signals. The gains may for example be implemented as a set of gains/ amplifiers that apply a variable gain to each analogue optical sensor signals where the variable gain can be set dynamically, e.g. via a suitable control signal.

The gain adjusted optical sensor signals are fed to an image generator 105 which is arranged to generate images from the gain adjusted optical sensor signals. The images are specifically a color image having color channels corresponding to the color channels of the sensors/ optical sensor signals, and thus are in the specific example RGB color images. The image generator 105 may generate the color channel values for one color channel by digitization of one of the optical sensor signals from one of the optical sensors. The image generator 105 may specifically comprise an analog to digital converter and perform a processing that generates video images from the digital samples of the optical sensor signals. Such a signal path of optical sensors, gain adjustments, and image generation is used in many optical cameras and may provide high quality captured images.

In the described approach, the camera apparatus further comprises a processor 107 which is fed the generated images. The processor 107 is arranged to determine vital signs based on the received video images. It is further arranged to adapt the capture operation and functionality in order to provide images that are particularly suitable for detecting vital signs. In particular, the processor 107 is arranged to determine gains for the optical sensor signals and control the gain circuit 103 to apply the determined gains. The gains may be determined individually for each color channel and different gains may be applied to the different optical sensor signals. It should be understood that the camera apparatus may be in one housing or may have components divided between multiple housings or, indeed, have components located in other pieces of equipment.

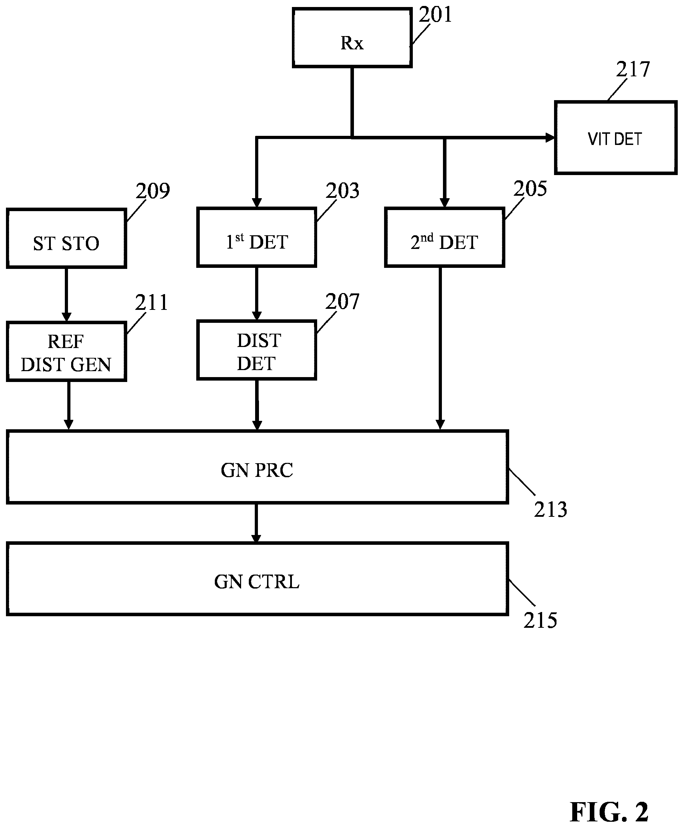

The processor 107 may apply a specific approach which will be described with reference to FIG. 2 which shows an example of elements of the processor 107.

The processor 107 comprises a receiver 201 which receives the video images from the image generator 105. Thus, the receiver 201 receives multi-color channel captured images that comprise a plurality of color channels, and specifically the images may be RGB video images.

The video images are fed to a first detector 203 and a second detector 205 which are arranged to detect different images areas in the received image(s).

The first detector 203 is arranged to detect a first image area in (at least one of) the received images/ frames which corresponds to a skin area of the person. The first detector 203 may thus analyze the image to detect an area which corresponds to an area that has captured the skin of the person. The first detector 203 may specifically in many embodiments perform face detection to detect a face of the person for which vital signs are determined.

It will be appreciated that many different approaches and algorithms are known and may be used for detecting a skin area.

For example, in some embodiments, the first image area may be determined as a substantially predetermined area corresponding to a fixed position of the user relative to the camera. For example, the camera may capture a scenario where the person is arranged to sit on a chair facing the camera and e.g. with the head fixed by a physical head bracket. In such a scenario, the face of the person will tend to always be at substantially the same position in the image, and the first image area may be determined as a predetermined area corresponding to the position of the face.

As another example, the first image area may include a face detection algorithm. It will be appreciated that many different face detection algorithms are known and that any suitable approach can be used without detracting from the invention. After detecting a position of a face in the image, the first detector 203 may proceed to generate the first image area as an area corresponding to the detected face position.

Thus, in many embodiments the first image area and the skin area may correspond to a face area of the person. However, it will be appreciated that in other embodiments, the first image area and skin area may alternatively or additionally be for another body part. For example, in some embodiments, the person may be required to put an arm in a specific position (e.g. supported by a restraint) that results in this being captured at a predetermined position in the image. As another example, a person may be required to be partially undressed and may be positioned such that the users upper body fills most of the image, and the first image area/ skin area may be determined as the largest area of the image for which the visual properties are substantially similar. For example, a segmentation based on image properties (e.g. color and luminance) may be performed for the image, and the first image area may be determined as the largest segment.

In many embodiments, the appropriate part of the body/ image segment may be tracked. For example, a person in bed may look around and the face tracker may be arranged to still focus on the face for camera control. As another example, a person may be moving in the room and the camera may track the person (and the face). The camera control may be arranged to not be impacted by other environmental factors (eg: for an example where a person walks in front of a window with sunlight - normally the camera would darken the entire video stream thereby losing the ability to monitor vital signs).

The second detector 205 is arranged to detect the second image area in the image(s). It may e.g. detect a predetermined area or may detect an image area based on the image properties. In many embodiments, the second image area may be determined as the image area which is used to determine the vital signs. For example, the processor 107 may be arranged to detect a pulse rate from the visual changes in a chest area of a person, and the second image area may therefore be selected to correspond to the chest area of the person.

In some embodiments, the second image area may be determined as a predetermined area in the video images, such as e.g. when the position of the person relative to the camera is tightly controlled. In other embodiments, it may e.g. be detected based on a marker or other recognizable visual characteristic in the image. For example, a marker with distinct visual properties may be attached to the person being imaged. The marker may e.g. surround an area of the body from which the vital signs are being determined. The second detector 205 may in this case detect an outline of the marker by detecting the specific visual properties (e.g. a specific pattern) in the image. It may then determine the second image area as the area within the determined outline.

As yet another example, the second image area may in some areas be determined as an area having a predetermined relationship to the first image area. For example, the second image area may be determined as a predetermined area located a predetermined distance below a detected face area and having a predetermined outline. This may for example allow a chest area to be determined from a face detection that determines the first image area.

As another example, the second image area may be determined by identifying a chest area using a body part detector.

The first detector 203 is coupled to a distribution determiner 207 which is arranged to determine a color channel distribution for pixels of the first image area. The color channel distribution may for each color channel generate a distribution of the pixel values for that color channel. The color channel distribution may for a given color channel indicate the frequency/ ratio of occurrence of pixel values of pixels belonging to the first image area. The color channel distribution may for each color channel indicate the occurrence or frequency of pixel values in the first image area for each (or groups) of the possible pixel values. For example, a histogram may be determined for each color channel reflecting the distribution of pixel values for that color channel.

The distribution determiner 207 may be arranged to generate a color channel distribution that for a given color channel and threshold value reflects the percentiles/ratio of pixel values that are below the threshold value (or alternatively equivalently above the threshold value). Equivalently, the distribution determiner 207 may be arranged to generate a color channel distribution that for a given color channel and percentile reflects a threshold value for which that percentile of pixel values is below the threshold value (or alternatively equivalently above the threshold value).

The camera apparatus further comprises a skin tone processor 209 which is arranged to provide a skin tone indication for the person being imaged and for which vital signs are determined. The skin tone indication is indicative of a skin tone of the person, and specifically of a visual skin tone property. In many embodiments, a plurality of skin tone categories may be stored by the skin tone processor 209 and the skin tone indication may indicate a category considered to be appropriate for the specific person being imaged.

In many cases, the assumption may be made that the ratio between the average RGB values for skin tissue is similar across skin phototypes, as found by De Haan, G., Jeanne, V., "Robust pulse-rate from chrominance-based rPPG". As will be described in detail later, such assumptions may be used to control the capture to provide images that are particularly suitable for detection of vital signs.

In some embodiments, the skin tone processor 209 may be coupled to or comprise a user input and the appropriate skin tone indication may be determined in response to a user input. For example, in some embodiments, a person may be presented with a set of images corresponding to the different skin type categories stored by the skin tone processor 209. The person may then select the image considered to most closely reflect the skin tone of the person being imaged and the skin tone indication may be set to indicate the corresponding category.

In some embodiments, the detection of a skin tone may e.g. be determined from an analysis of the captured video images and specifically the first image area. An average pixel value may for example be determined for each color channel and compared to a set of reference average pixel values for each of the skin tone categories. The skin tone category with the closest matching reference average values may then be indicated by the skin tone indication to be the skin tone of the person. Although such a detection may sometimes be quite inaccurate due to it being based on initial images that may not be captured with the captured process being fully adapted or optimized, it will typically be sufficient as the differences between the reference average values may be quite substantial.

The skin tone processor 209 is coupled to a reference processor 211 which is arranged to provide a reference color channel distribution property for the indicated skin tone. Thus, based on the received skin tone indication, the reference processor 211 may determine a reference distribution property for each of the color channels. The property may in different embodiments be different properties representing a property or characteristic of the distribution (typically individually in each color channel). For example, the property may be an indication of an average, a variance, a percentile, a mode, a median. Specifically, in many embodiments, the reference property may be a set of pixel values corresponding to a given (e.g. predetermined) percentile of the distribution of pixel values in a color channel.

The reference color channel distribution property may specifically comprise a set of reference intensity values for the different color channels. For example, a pixel value may be provided for each color channel. The reference pixel value may for example indicate a preferred pixel value for a given percentile of the distribution of pixel values in the first image area.

The reference processor 211 may in many embodiments store a set of reference properties, such as reference intensity values, for each of the possible skin tone categories. The reference properties may for example be predetermined values that are e.g. stored in memory during a manufacturing process. Thus, predetermined stored reference values may in some embodiments be associated with each possible skin tone category. The appropriate values may for example have been found during testing and experimentation during development and design of the approach to determine vital signs. The reference properties may specifically have been optimized to result in the desired, and as far as possible the optimal, detection performance for the vital sign determination.

The reference processor 211 is coupled to a gain processor 213 which is arranged to determine a gain for the gain circuit 103 based on the reference color channel distribution property provided for the specific skin tone and on the color channel distribution for pixels of the first image area. The gain processor 213 may specifically adapt gains for the color channels to reduce a difference between the reference color channel distribution property and a property for the color channel distribution. Thus, the gain processor 213 may be arranged to determine gains to reduce a difference between a property of the determined color channel distribution and the reference color channel distribution property for the specific skin tone indicated for the person. Specifically, it may seek to set the gains to reduce the difference between the reference intensity values for each color channel and determined intensity values for a given percentile for each of the color channel distributions.

For example, the reference color channel distribution property may indicate a reference pixel/ intensity value for each color channel. The reference pixel value may for example indicate a preferred pixel value for a given percentile of pixels (say the 90th percentile). The gain processor 213 may for each color channel determine the pixel value for the 90th percentile for the specific determined color channel distribution for the first image area. The gain may then for each color channel be set to the ratio between the preferred pixel value and the determined 90^{th} percentile pixel value. Thus, if the pixel values of the individual pixels of the first image area were scaled by the determined gain value, it would result in the first image area having a 90^{th} percentile value corresponding to the preferred value. Accordingly, applying the determined gain to the gain circuit 103 will result in captured images having the desired properties.

However, the gain processor 213 is further arranged to not only consider the first image area when determining the gains but rather the gain determination is also dependent on properties of the second image area. The gain processor 213 is specifically arranged to determine a luminance property for the second image area and to set the gains in response to this luminance property. In many embodiments, the luminance property may be a gain compensated luminance property, and may reflect a luminance for the second image area that would have resulted if the determined gains had been applied to the optical sensor signals, and thus which will result for future images unless the capture environment changes. For example, the luminance property may represent the luminance of the second image area that will result from setting the gains for the optical sensor signals as determined by the gain processor 213 and with no other changes to the capture or lighting conditions.

In many cases, a luminance property may be determined for the second image area and then scaled by a factor that is dependent on the determined gains. For example, for a given gain setting, a predetermined function may be evaluated to provide a gain reflecting how the luminance will be modified by a change of the gains from those that were used when the image was captured to those that are currently considered. The luminance property may then be scaled by this value to provide a gain compensated luminance.

The luminance property may be indicative of a luminance level for the second image area, and specifically may be an indication of a maximum, average, median, percentile (e.g. 90^{th} percentile) luminance level for the second image area. The luminance property typically indicates the luminance level as a luminance per area (e.g. per pixel) relative to a luminance range. For example, the range may have a lowest luminance level corresponding to all color channel values having a minimum value (e.g. a RGB values of (0,0,0)) and a highest luminance level corresponding to all color channel values having a maximum value (e.g. a RGB values of (255,255,255) for 8 bit values).

As a low complexity example, a luminance value may be calculated for each pixel of the second image area in accordance with a suitable luminance measure (e.g. by combining the individual color channel values following a weighting by a factor reflecting the relative contribution/ significance of each color channel when determining vital signs). An average, mean, median, or e.g. a given percentile (say 90^{th} percentile) luminance value may then be determined.

The gain processor 213 may then adapt the gains taking the luminance property into account. For example, the previously described gain determination may be applied to set the 90^{th} percentile color channel pixel values to the preferred values. However, rather than blindly using these gain values, they may be made subject to a requirement that the luminance property for the second image area must meet a given criterion. For example, a luminance range may be given by a predetermined minimum luminance level and a predetermined maximum luminance level. The gain values for the individual color channels may be determined as previously described. A corresponding luminance gain may be determined (weighting the gains of the individual color channels appropriately to reflect their impact on the luminance) to generate a gain compensated luminance property. For example, the luminance property (e.g. the 90^{th} percentile luminance) for the second image area may be multiplied by the luminance gain and the resulting value may be compared to the luminance range. If the modified luminance is below the predetermined range, the gain values may be adapted (increased) until the modified luminance exceeds the predetermined minimum luminance value. Similarly, if the modified luminance is above the predetermined range, the gain values may be adapted (decreased) until the modified luminance is below the predetermined maximum luminance value.

The operations may in many embodiments be linear and the adaptations of the gain to ensure the luminance property of the second image area is within the desired range may typically be achieved by scaling the gains determined based on the color channels of the first image area, with the scaling being the same for all color channels. Thus, the determined gains may simply be multiplied by a given factor that is the same for all color channels. In many cases, the scale factor may simply be the ratio between the predetermined minimum luminance value and the determined luminance value (if below the minimum luminance value) or may be the ratio between the predetermined maximum luminance value and the determined luminance value (if above the maximum luminance value).

The gain processor 213 is coupled to a gain controller 215 which is fed the determined gains, and which is arranged to control the gain circuit 103 (and in some cases the exposure properties of the sensors 101) to apply the determined gains. For example, the gain processor 213 may be arranged to set amplification levels of gain amplifiers of the gain circuit 103 to match the determined gains.

The approach may thus provide a feedback mechanism that adjusts the capture operation to provide images that have a color channel balance that can be carefully controlled to provide advantageous properties for detecting vital signs. This is achieved by the specific consideration of skin tone thereby allowing not only an efficient and reliable adaptation and calibration but also allows for a specific adaptation and potential optimization for the individual person. Further, the advantageous color channel balancing is achieved while at the same time providing adaptation and ensuring that imaging of a given image area that typically is used to determine the vital signs is suitable and advantageous for such detection. The approach may advantageously consider different areas of the person and by a combined adaptation/ calibration ensure that imaging is done so that both advantageous color balancing (which in many cases can be considered to be a white balance operation) and luminance adaptation can be achieved and optimized for vital sign detection.

The camera apparatus comprises a vital sign detector 217 which is arranged to detect vital signs based on the video images. After an initial gain setting, the video images may specifically be adapted and possibly optimized for the specific vital sign detection algorithm as previously described. For example, color channels may be adapted individually to highlight features that are specifically suitable for detecting specific properties. As an example, in some cases, the variation in the captured image of a person due to a pulse may be more pronounced in some color channels than others. For example, depending on the skin tone, the optical variations for the given lighting of the person may be different in different color channels. The described approach may weigh the color channels for which the variation is higher relative to color channels for which variation is lower.

The vital sign detector 217 is fed the video images and performs a suitable image based vital sign detection/ algorithm.

For example, the vital sign detector 217 may perform a photoplethysmography algorithm to determine volumetric variations of blood circulation from the video images. Such measurements may specifically be used to determine a pulse rate. Specifically, the effect of volumetric variations (the light absorption by blood) on the spectrum of the light may be assumed identical between persons; its direction in RGB color space shows little variation between persons. Measurement of the pulse signal can be considered a least-squares optimization problem given the knowledge of the pulse color direction. As the skin is in fact a person-dependent filter that changes the spectrum of the light source, the color signals are normalized using a short-time averaging operation prior to the least-square optimization, in order to compensate for the effect of the skin.

The effect of volumetric variations (the light absorption by blood) on the spectrum of the light is assumed identical between persons; its direction in RGB color space shows little variation between persons. Measurement of the pulse signal can be considered a least-squares optimization problem given the knowledge of the pulse color direction. As the skin is in fact a person-dependent filter that changes the spectrum of the light source, the color signals are normalized using a short-time averaging operation prior to the least-square optimization, in order to compensate for the effect of the skin.

As another example, the vital sign detector 217 may determine a respiration signal by cross-correlating a one-dimensional representation of an image (or a selected region of interest of that image) with that of an earlier image. This one-dimensional representation can be any function that captures the texture that in the image or region-of-interest (for example, by calculating the mean over all the pixel values in a row). The maximum correlation is a derivative of the chest position and when integrated, forms a measure of chest expansion (respiration signal) during breathing. And all derivatives of pulse and respiration eg: interbeat interval, respiratory effort etc.

As another example, the vital sign detector 217 may determine a pulse transit time based on the extracted pulse signal from the skin at two different body locations (or skin at one location and ECG). This can be used as a surrogate for blood pressure (Ref e.g.

### https://pubmed.ncbi.nlm.nih.gov/28324936/).

Different algorithms and functions for determining the gains based on the reference color channel distribution property, the property of the set of color channel distributions, and the luminance property may be used in different embodiments.

As described, in many embodiments, the gain setting may mainly be determined based on a consideration of color channel intensity values and the distribution of these in the individual color channels relative to the reference intensity values for the color channels. The color channel intensity values may be represented by the color channel pixel values, e.g. each of the RGB values.

The gains may in some embodiments be determined as a function of each of the values. For example, the gain processor 213 may comprise a Look-Up-Table (LUT) which as an input has the luminance value determined for the second image area, a given statistical property for each color channel describing each color channel distribution (e.g. a 90^{th} percentile pixel value), and the reference intensity values for the different color channels. The LUT may, based on these input values, provide stored values for a set of gain values for the color channels. Thus, the LUT may directly provide appropriate gain settings.

Such a LUT may for example be determined by a performing a large number of tests during a design phase and determining gain values that provide an efficient vital sign detection for different parameters, settings, and scenarios. The LUT may be populated by gain values determined in this way. In other examples, gain values may be determined based on e.g. part measurements and/or analytical evaluations.

In some embodiments, such approaches may instead of populating a LUT be used to determine a functional relationship between the different parameters and suitable gain values, and the gain processor 213 may be arranged to apply such functions to determine suitable gains.

In many embodiments, the gain processor 213 may be arranged to determine individual gain values for each of the color channels based on a reference intensity value and a determined intensity value for that color channel. The gain processor 213 may determine a gain for a first color channel in dependence on a difference between a determined intensity value for the first color channel and a reference intensity value for the first color channel value. For example, as described previously, in some embodiments, the gain may be determined to reflect the difference between the determined intensity value and the reference intensity value for the given color channel. Specifically, the gain for a given color channel may be determined as the ratio between the reference intensity value and the determined intensity value. For example, the reference intensity value may indicate a preferred 90^{th} percentile color channel value and the determined intensity value may indicate a measured 90^{th} percentile color channel value for the first image area. Determining the gain as the ratio between these values will adapt the capture operation such that the measured 90^{th} percentile value for the image area is set to the preferred value.

Further, applying this approach individually to all color channels will result in all color channels having the desired scaling. Applying this approach, to all the color channels will furthermore achieve that the relationship, and specifically the ratio, between determined intensity values for the different color channels match the relationship, and specifically the ratio, between reference intensity values for the different color channels. This may provide particularly advantageous operation in many scenarios, and may typically allow particular color channels to be emphasized, or indeed may allow a white balance of the images to be set as desired (including as natural as possible).

Indeed, in many embodiments, the gains may generally be generated to maintain the same color channel relationship as the reference intensity values without the individual color channels necessarily being set to the reference intensity values. For example, based on the luminance property for the second image area, a common gain component for all the gains may be varied e.g. to ensure that the luminance property remains within a given range. This may result in the individual color channel properties being different from the reference values but with the ratio/ relationship between the color channels being maintained.

In many embodiments, individual gain components are determined for the color channels based on the determined intensity values and the reference intensity values, and a common gain component for all color channels is determined based on the luminance property. For example, the gain values for the individual color channels may be determined as described above based on the reference intensity values and determined intensity values of the individual color channel values. The gain processor 213 may then for these gains determine a gain compensated luminance property that corresponds to the measured luminance value for the second image area scaled in proportion to the determined gains. If the resulting gain compensated luminance value is within a given range, the gain values may be maintained and otherwise a common scale factor for all color channels may be applied to the determined gains to result in the gain compensated luminance value falling within the given range.

In some embodiments, the gain processor 213 is arranged to control an exposure property of the optical sensors in dependence on the luminance property and/or on the comparison of the reference color channel distribution property and a property of the set of color channel distributions. The exposure property may specifically be a shutter speed/ duration and/or an aperture. Thus, the gain controller 215 may in some embodiments also be arranged to control the capture operation of the optical sensors. These exposure properties may control how much light is captured for each image. E.g. increasing the shutter duration and/or the aperture results in more light reaching the sensor and thus in larger values of the optical sensor signals and accordingly in higher pixel values.

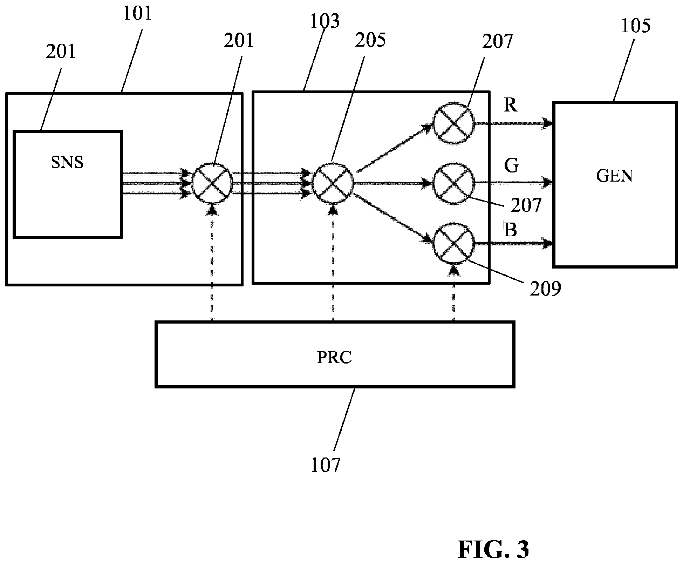

Indeed, typically, the effect of changing the exposure control has the same effect as changing a gain/ amplification applied to the optical sensor signals, and indeed it can be modelled as such. The signal path of the capture operation can accordingly be illustrated by the example of FIG. 3 where gain 201 represents the effect of changing the exposure parameters of the sensors 201. Gain 205 represents a main gain that is common to all the color channels, and gains 207 represent gains that are individual to each color channel.

Thus, for a given set of determined gains, these may be separated into a common gain and individual gains for each color channel. For example, the common gain is applied to all the optical sensor signals and may e.g. be set to the gain value determined for one of the color channels. The individual gain for that color channel may then be set to 1 and the individual gains for the other color channel gains may be set to the ratio between the determined gain for that color channel and the common gain.

The common gain may be implemented by controlling the exposure and setting the main gain. The exact approach may depend on the specific preferences and requirements of the individual embodiment. For example, in some embodiments, it may be desirable to set the exposure properties to maximize the light capture (high aperture, long exposure) in order to maximize the signal to noise ratio. The remaining common gain may then be implemented by the main gain. In other examples, it may be desirable to set the shutter speed and/or aperture to desired values (or within a range) to provide a sharp image. The main gain may then again be set to result in the desired overall common gain.

As a specific example, the following approach may be used to implement the determined gains:
1. The smallest gain may be implemented using the exposure and master gain, i.e. the common gain is set to the lowest determined color channel gain.
2. The gain controller 215 may first maximize the exposure, i.e. it may set the shutter speed and/or aperture to a maximum value to improve the signal-to-noise ratio. The remaining contribution to the common gain is then implemented by setting the main gain appropriately. In many embodiments, the master gain may only be settable with quite coarse granularity so the nearest quantization step may be set. The following steps may be performed to further fine-tune the capture operation:
3. The gain controller 215 may for the determined exposure and main gain settings determine the corresponding total gain for a given color channel. This may specifically be done for a single color channel, and typically for the green color channel as this has the highest contribution to luminance and is most important for blood volume pulse measurement. Because the master gain is generally coarse, the resulting multiplication factor will generally not match the desired multiplication factor for the green color channel.
4. In order to fine tune the gain for the green color channel, a fine-tuning of the exposure properties may be performed. Thus, the shutter speed and/or the aperture may be adjusted to more accurately achieve the overall desired gain for the green color channel.
5. The individual color channel gains for the red and blue color channels may then be determined for the given exposure and master gain settings that have been determined for the green color channel.

The above approach may in many embodiments be applied to all images. However, in many embodiments, the camera apparatus may be arranged to differentiate between images for which a skin area is/can be detected and images for which no skin area is/ can be detected. Specifically, for embodiments being based on face detection to determine the first image area, the system may be arranged to differentiate between images for which the face detection is successful and images for which the face detection is not successful.

The gain processor 213 may specifically in such embodiments be arranged to determine color channel distributions for images in which a skin area is detected and to store these for use when determining gains for images for which no skin area is detected. The color channel distributions that are determined and stored are typically for different areas than the first and second area, and specifically are typically larger. In many embodiments, the gain processor 213 determines a color channel distribution for the whole image in which a skin area has been detected and stores this full image color channel distribution.

When an image is being processed for which no skin area has been detected, the gain processor 213 may proceed to retrieve the stored color channel distribution and may further determine a color channel distribution for the current image (also for conciseness referred to as a no-skin image). The color channel distribution for the no-skin image (also for conciseness referred to as a current color channel distribution) may be determined for the same image area for which the stored color channel distribution is determined, and typically it may be determined for a full image, i.e. the image area for which the current color channel distribution may be determined as the full image area.

As a low complexity example, if the comparison indicates that the stored color channel distribution and the current color channel distribution are sufficiently similar (in accordance with any suitable difference/ similarity criteria), the gain processor 213 may proceed to directly reuse the gains that were determined for the image of the stored color channel distribution (and thus these gains may have been stored with the color channel distribution). If the distributions are not sufficiently similar, the gain processor 213 may e.g. proceed to instead apply nominal or default gain values.

In many embodiments, the gain processor 213 may be arranged to modify the gains determined for the stored color channel distribution to reflect the difference between this distribution and the current color channel distribution. The compensation may specifically be such that the resulting difference between the color channel distributions is reduced.

For example, the gain processor 213 may determine a given (say the 90^{th}) percentile intensity/ pixel value for each color channel of both distributions. A compensation factor may then for each color channel be determined as the ratio between the (90^{th}) percentile value of the stored color channel distribution and the (90^{th}) percentile value of the current color channel distribution. The gain for each color channel may then be determined as the stored gain for the stored color channel distribution multiplied by the compensation factor. This may result in gain settings that will achieve the same (90^{th}) percentile values which may be suitable for many practical scenarios for which the changes in the capture environment and the time intervals in which face detection occurs are relatively small.

As a specific example, in some embodiments, the gain processor 213 may be arranged to determine full-image percentile intensity values for all images, color channels, and percentiles (0 to 100 percent, for each color channel). These values may be stored (i.e. a color channel distribution given by the percentile values is stored). If a situation then arises where no skin area is detected, the gain processor 213 may retrieve the stored color channel distribution of the last image in which a skin area was detected. It may then determine the percentile for which all percentiles are equal or below the highest target percentile for the first image area. This percentile may then be used as the new target value for the channel having the highest intensity value for the given percentile.

Such an approach may for example allow that if no skin area is detected, the capture operation remains unchanged unless there is a considerable illumination change in which case the gain processor 213 will seek to reproduce the conditions as they were just before the failure to detect the skin area.

A particular issue in some embodiments may be the start-up process and how to set the initial capture properties. In particular, it may be highly desirable to set initial gain values such that suitable images are captured, and in many embodiments such that accurate skin/ face detection can be achieved.

In some embodiments, the gain processor 213 may be arranged to determine initial gains based on a consideration of a property of the full image, and thus without performing or requiring skin or face detection. The gain processor 213 may analyze a full image to determine an intensity value/ property. For example, the gain processor 213 may for each color channel determine a given percentile intensity value. For example, the pixel/ intensity value for, say, the 95th percentile of the entire image may be determined for each color channel. The determined pixel/ intensity value may then be compared to a predetermined reference intensity value which specifically may be set to reflect a preferred intensity value for the 95th percentile. The predetermined reference intensity value may e.g. represent a value of the 95th percentile which in suitable trials and analyses has been found to typically provide a suitable exposure setting resulting in images that in particular may be suitable for skin area and/or face detection. A common initial gain for all color channels may in some embodiments be determined, and in particular may be set to the ratio between the predetermined reference intensity value and the determined percentile intensity value.

In some embodiments, the percentile intensity value may be determined for only one of the color channels. For example, one color channel may be more significant for face detection and the exposure may be optimized for that color channel. However, in many embodiments, values may be determined for all color channels and the percentile intensity value that is compared to the predetermined reference intensity value may be selected as the highest value for each of the color channels. Accordingly, the initial gains may be adjusted such that the given percentile value for the highest intensity color channel is set to a preferred level. This may in many scenarios allow for images to be captured that are suitable for skin/ face detection.

As a specific example, the gain processor 213 may perform initial full-image exposure control to enable initial face detection and tracking. The initial camera control may control the maximum measured full-image nth percentile towards a (configurable) target value.

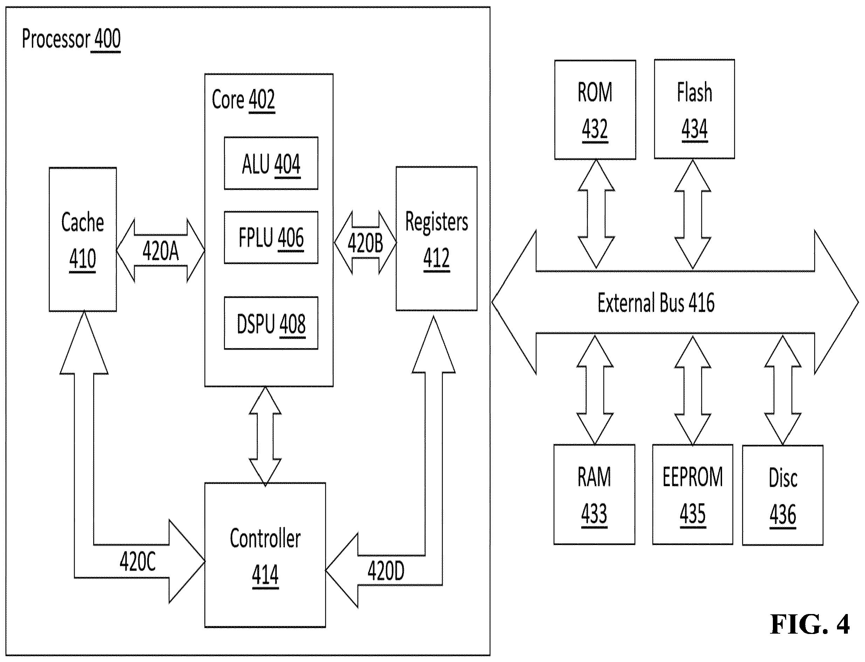

FIG. 4 is a block diagram illustrating an example processor 400 according to embodiments of the disclosure. Processor 400 may be used to implement one or more processors implementing an apparatus as previously described or elements thereof. Processor 400 may be any suitable processor type including, but not limited to, a microprocessor, a microcontroller, a Digital Signal Processor (DSP), a Field ProGrammable Array (FPGA) where the FPGA has been programmed to form a processor, a Graphical Processing Unit (GPU), an Application Specific Integrated Circuit (ASIC) where the ASIC has been designed to form a processor, or a combination thereof.

The processor 400 may include one or more cores 402. The core 402 may include one or more Arithmetic Logic Units (ALU) 404. In some embodiments, the core 402 may include a Floating Point Logic Unit (FPLU) 406 and/or a Digital Signal Processing Unit (DSPU) 408 in addition to or instead of the ALU 404.

The processor 400 may include one or more registers 412 communicatively coupled to the core 402. The registers 412 may be implemented using dedicated logic gate circuits (e.g., flip-flops) and/or any memory technology. In some embodiments the registers 412 may be implemented using static memory. The register may provide data, instructions and addresses to the core 402.

In some embodiments, processor 400 may include one or more levels of cache memory 410 communicatively coupled to the core 402. The cache memory 410 may provide computer-readable instructions to the core 402 for execution. The cache memory 410 may provide data for processing by the core 402. In some embodiments, the computer-readable instructions may have been provided to the cache memory 410 by a local memory, for example, local memory attached to the external bus 416. The cache memory 410 may be implemented with any suitable cache memory type, for example, Metal-Oxide Semiconductor (MOS) memory such as Static Random Access Memory (SRAM), Dynamic Random Access Memory (DRAM), and/or any other suitable memory technology.

The processor 400 may include a controller 414, which may control input to the processor 400 from other processors and/or components included in a system and/or outputs from the processor 400 to other processors and/or components included in the system. Controller 414 may control the data paths in the ALU 404, FPLU 406 and/or DSPU 408. Controller 414 may be implemented as one or more state machines, data paths and/or dedicated control logic. The gates of controller 414 may be implemented as standalone gates, FPGA, ASIC or any other suitable technology.

The registers 412 and the cache 410 may communicate with controller 414 and core 402 via internal connections 420A, 420B, 420C and 420D. Internal connections may be implemented as a bus, multiplexer, crossbar switch, and/or any other suitable connection technology.

Inputs and outputs for the processor 400 may be provided via a bus 416, which may include one or more conductive lines. The bus 416 may be communicatively coupled to one or more components of processor 400, for example the controller 414, cache 410, and/or register 412. The bus 416 may be coupled to one or more components of the system.

The bus 416 may be coupled to one or more external memories. The external memories may include Read Only Memory (ROM) 432. ROM 432 may be a masked ROM, Electronically Programmable Read Only Memory (EPROM) or any other suitable technology. The external memory may include Random Access Memory (RAM) 433. RAM 433 may be a static RAM, battery backed up static RAM, Dynamic RAM (DRAM) or any other suitable technology. The external memory may include Electrically Erasable Programmable Read Only Memory (EEPROM) 435. The external memory may include Flash memory 434. The External memory may include a magnetic storage device such as disc 436. In some embodiments, the external memories may be included in a system.

The invention can be implemented in any suitable form including hardware, software, firmware, or any combination of these. The invention may optionally be implemented at least partly as computer software running on one or more data processors and/or digital signal processors. The elements and components of an embodiment of the invention may be physically, functionally and logically implemented in any suitable way. Indeed the functionality may be implemented in a single unit, in a plurality of units or as part of other functional units. As such, the invention may be implemented in a single unit or may be physically and functionally distributed between different units, circuits and processors.

In the application any reference to one of the terms "in response to", "based on", "in dependence on", and "as a function of" should be considered to be a reference to the term "in response to /based on/ in dependence on/ as a function of". Any of the terms should be considered to be a disclosure of any of the other terms and the use of only a single term should be considered a short-hand notation that includes the other alternatives/ terms.

Although the present invention has been described in connection with some embodiments, it is not intended to be limited to the specific form set forth herein. Rather, the scope of the present invention is limited only by the accompanying claims.

## Claims

1. An apparatus for estimating vital signs of a person from video images, the apparatus comprising:
a receiver (201) arranged to receive video images comprising a plurality of color channels, each color channel representing a digitization of an optical sensor signal from a color channel optical sensor;
a first detector (203) arranged to determine a first image area in at least one image of the video images, the first image area corresponding to a skin area of the person;
a second detector (205) arranged to determine a second image area in at least one image of the video images, the second image area corresponding to a different area of the person than the first image area;
a determiner (207) arranged to determine a set of color channel distributions for pixels of the first image area;
a processor (209) arranged to provide a skin tone indication, the skin tone indication being indicative of a skin tone of the person;
a reference processor (211) arranged to provide a reference color channel distribution property for the skin tone;
a gain processor (213) arranged to determine gains for the optical sensor signals in dependence on a luminance property determined for the second image area and in dependence on a difference between the reference color channel distribution property and a property of the set of color channel distributions, wherein the determined gains are adapted to reduce the difference;
a gain controller (215) arranged to control gain values applied to the optical sensor signals to match the determined gains; and
a vital sign determiner (217) arranged to estimate a vital sign property for the person from the video images.

2. The apparatus of claim 1 further comprising:
a gain circuit (103) arranged to apply the gain values to optical sensor signals from at least two color channel optical sensors; and
an image generator (105) coupled to the gain circuit (103) and arranged to generate the video images from the optical sensor signals received from the gain circuit (103).

3. The apparatus of any previous claim wherein the reference color channel distribution property comprises a set of reference intensity values for different color channels, and the property of the color channel distribution comprises a set of determined intensity values for the set of color channel distributions.

4. The apparatus of claim 3 wherein the determined intensity values are color channel pixel values for a given percentile of the set of color channel distributions.

5. The apparatus of claim 3 or 4 wherein the gain processor (213) arranged to determine a gain for a first color channel of the plurality of color channels in dependence on a difference between a determined intensity value for the first color channel and a reference intensity value for the first color channel value.

6. The apparatus of any of claims claim 3 to 5 wherein the gain processor (213) arranged to set the gains such that a relationship between determined intensity values for the different color channels match a relationship between reference intensity values for the different color channels.

7. The apparatus of any of claims claim 3 to 5 wherein the luminance property is a luminance for the second image area resulting from applying the determined gains to the second image area, and the gain processor (213) is arranged to set the gains subject to the luminance property being indicative of a luminance for the second image area meeting a criterion.

8. The apparatus of any of the previous claims wherein the gain processor (213) is arranged to determine a common gain component in dependence on the luminance property and relative gain components in dependence on the comparison of the reference color channel distribution property and a property of the set of color channel distributions, the common gain component being a gain applied to all color channels of the plurality of color channels and the relative gain components being gain components applied to individual color channels of the plurality of color channels.

9. The apparatus of any previous claim wherein the gain processor (213) is arranged to control an exposure property of at least one color channel optical sensor in dependence on at least one of the luminance property and the comparison of the reference color channel distribution property and a property of the set of color channel distributions.

10. The apparatus of any previous claim wherein the gain processor (213) is arranged to determine and store a first color channel distribution for a third image area of an image for which a skin area has been detected; and to determine gains for the optical sensor signals for a first image for which no skin area has been detected in dependence on the first color channel distribution and a second color channel distribution being determined for a third image area for the first image.

11. The apparatus of claim 10 wherein the gain processor (213) is arranged to set the gain to reduce a difference between a property of the first color channel distribution and the second color channel distribution.

12. The apparatus of claim 10 or 11 wherein the third image area is a full image area.

13. The apparatus of any previous claim wherein the gain processor (213) is arranged to determine initial gains for the optical sensor signals in dependence on comparison of a full image intensity value to a predetermined reference intensity value; the full image intensity value being an intensity value for a predetermined percentile of a color channel of the plurality of color channels.

14. A computer-implemented method of estimating vital signs of a person from images, the method comprising
receiving video images comprising a plurality of color channels, each color channel representing a digitization of an optical sensor signal from a color channel optical sensor;
determining a first image area in at least one image of the video images, the first image area corresponding to a skin area of the person;
determining a second image area in at least one image of the video images, the second image area corresponding to a different area of the person than the first image area;
determining a set of color channel distributions for pixels of the first image area;
providing a skin tone indication, the skin tone indication being indicative of a skin tone of the person;
providing a reference color channel distribution property for the skin tone;
determining gains for the optical sensor signals in dependence on a luminance property determined for the second image area and in dependence on a difference between the reference color channel distribution property and a property of the set of color channel distributions, wherein the determined gains are determined so as to reduce the difference;
controlling gain values applied to the optical sensor signals to match the determined gains; and
estimating a vital sign property for the person from the video images.

15. A computer program product comprising computer program code means adapted to perform all the steps of claim 14 when said program is run on a computer.

## Patentansprüche

1. Einrichtung zum Schätzen von Vitalzeichen einer Person aus Videobildern, wobei die Einrichtung Folgendes umfasst:
einen Empfänger (201), der dazu ausgelegt ist, Videobilder zu empfangen, die mehrere Farbkanäle umfassen, wobei jeder Farbkanal eine Digitalisierung eines optischen Sensorsignals aus einem optischen Farbkanalsensor repräsentiert;
einen ersten Detektor (203), der dazu ausgelegt ist, einen ersten Bildbereich in mindestens einem Bild der Videobilder zu bestimmen, wobei der erste Bildbereich einem Hautbereich der Person entspricht;
einen zweiten Detektor (205), der dazu ausgelegt ist, einen zweiten Bildbereich in mindestens einem Bild der Videobilder zu bestimmen, wobei der zweite Bildbereich einem anderen Bereich der Person als dem ersten Bildbereich entspricht;
einen Bestimmer (207), der dazu ausgelegt ist, einen Satz von Farbkanalverteilungen für Pixel des ersten Bildbereichs zu bestimmen;
einen Prozessor (209), der dazu ausgelegt ist, eine Hauttonangabe bereitzustellen, wobei die Hauttonangabe einen Hautton der Person angibt;
einen Referenzprozessor (211), der dazu ausgelegt ist, eine Referenzfarbkanalverteilungseigenschaft für den Hautton bereitzustellen;
einen Verstärkungsprozessor (213), der dazu ausgelegt ist, Verstärkungen für die optischen Sensorsignale in Abhängigkeit von einer für den zweiten Bildbereich bestimmten Luminanzeigenschaft und in Abhängigkeit von einer Differenz zwischen der Referenzfarbkanalverteilungseigenschaft und einer Eigenschaft des Satzes von Farbkanalverteilungen zu bestimmen, wobei die bestimmten Verstärkungen dazu ausgelegt sind, die Differenz zu reduzieren;
eine Verstärkungssteuerung (215), die dazu ausgelegt ist, Verstärkungswerte, die auf die optischen Sensorsignale angewendet werden, so zu steuern, dass sie mit den bestimmten Verstärkungen übereinstimmen; und
einen Vitalzeichenbestimmer (217), der dazu ausgelegt ist, eine Vitalzeicheneigenschaft für die Person aus den Videobildern zu schätzen.

2. Einrichtung nach Anspruch 1, die ferner Folgendes umfasst:
eine Verstärkungsschaltung (103), die dazu ausgelegt ist, die Verstärkungswerte auf optische Sensorsignale aus mindestens zwei optischen Farbkanalsensoren anzuwenden; und
einen Bildgenerator (105), der mit der Verstärkungsschaltung (103) gekoppelt ist und dazu ausgelegt ist, die Videobilder aus den optischen Sensorsignalen zu erzeugen, die aus der Verstärkungsschaltung (103) empfangen werden.

3. Einrichtung nach einem der vorhergehenden Ansprüche, wobei die Referenzfarbkanalverteilungseigenschaft einen Satz von Referenzintensitätswerten für unterschiedliche Farbkanäle umfasst und die Eigenschaft der Farbkanalverteilung einen Satz von bestimmten Intensitätswerten für den Satz von Farbkanalverteilungen umfasst.

4. Einrichtung nach Anspruch 3, wobei die bestimmten Intensitätswerte Farbkanalpixelwerte für ein gegebenes Perzentil des Satzes von Farbkanalverteilungen sind.

5. Einrichtung nach Anspruch 3 oder 4, wobei der Verstärkungsprozessor (213) dazu ausgelegt ist, eine Verstärkung für einen ersten Farbkanal der mehreren Farbkanäle in Abhängigkeit von einer Differenz zwischen einem bestimmten Intensitätswert für den ersten Farbkanal und einem Referenzintensitätswert für den ersten Farbkanalwert zu bestimmen.

6. Einrichtung nach einem der Ansprüche 3 bis 5, wobei der Verstärkungsprozessor (213) dazu ausgelegt ist, die Verstärkungen so einzustellen, dass eine Beziehung zwischen bestimmten Intensitätswerten für die verschiedenen Farbkanäle mit einer Beziehung zwischen Referenzintensitätswerten für die verschiedenen Farbkanäle übereinstimmt.

7. Einrichtung nach einem der Ansprüche 3 bis 5, wobei die Luminanzeigenschaft eine Luminanz für den zweiten Bildbereich ist, die sich aus dem Anwenden der bestimmten Verstärkungen auf den zweiten Bildbereich ergibt, und der Verstärkungsprozessor (213) dazu ausgelegt ist, die Verstärkungen abhängig davon einzustellen, dass die Luminanzeigenschaft angibt, dass eine Luminanz für den zweiten Bildbereich ein Kriterium erfüllt.

8. Einrichtung nach einem der vorhergehenden Ansprüche, wobei der Verstärkungsprozessor (213) dazu ausgelegt ist, eine gemeinsame Verstärkungskomponente in Abhängigkeit von der Luminanzeigenschaft und relative Verstärkungskomponenten in Abhängigkeit von dem Vergleich der Referenzfarbkanalverteilungseigenschaft und einer Eigenschaft des Satzes von Farbkanalverteilungen zu bestimmen, wobei die gemeinsame Verstärkungskomponente eine Verstärkung ist, die auf alle Farbkanäle der mehreren Farbkanäle angewendet wird, und die relativen Verstärkungskomponenten Verstärkungskomponenten sind, die auf einzelne Farbkanäle der mehreren Farbkanäle angewendet werden.

9. Einrichtung nach einem der vorhergehenden Ansprüche, wobei der Verstärkungsprozessor (213) dazu ausgelegt ist, eine Belichtungseigenschaft von mindestens einem optischen Farbkanalsensor in Abhängigkeit von der Luminanzeigenschaft und/oder dem Vergleich der Referenzfarbkanalverteilungseigenschaft und einer Eigenschaft des Satzes von Farbkanalverteilungen zu steuern.

10. Einrichtung nach einem der vorhergehenden Ansprüche, wobei der Verstärkungsprozessor (213) dazu ausgelegt ist, eine erste Farbkanalverteilung für einen dritten Bildbereich eines Bildes, für den ein Hautbereich detektiert wurde, zu bestimmen und zu speichern; und Verstärkungen für die optischen Sensorsignale für ein erstes Bild, für das kein Hautbereich detektiert wurde, in Abhängigkeit von der ersten Farbkanalverteilung und einer zweiten Farbkanalverteilung, die für einen dritten Bildbereich für das erste Bild bestimmt wird, zu bestimmen.

11. Einrichtung nach Anspruch 10, wobei der Verstärkungsprozessor (213) dazu ausgelegt ist, die Verstärkung so einzustellen, dass eine Differenz zwischen einer Eigenschaft der ersten Farbkanalverteilung und der zweiten Farbkanalverteilung reduziert wird.

12. Einrichtung nach Anspruch 10 oder 11, wobei der dritte Bildbereich ein Vollbildbereich ist.

13. Einrichtung nach einem der vorhergehenden Ansprüche, wobei der Verstärkungsprozessor (213) dazu ausgelegt ist, anfängliche Verstärkungen für die optischen Sensorsignale in Abhängigkeit von einem Vergleich eines Vollbildintensitätswerts mit einem vorbestimmten Referenzintensitätswert zu bestimmen; wobei der Vollbildintensitätswert ein Intensitätswert für ein vorbestimmtes Perzentil eines Farbkanals der mehreren Farbkanäle ist.

14. Computerimplementiertes
Verfahren zum Schätzen von Vitalzeichen einer Person aus Bildern, wobei das Verfahren umfasst:
Empfangen von Videobildern, die mehrere Farbkanäle umfassen, wobei jeder Farbkanal eine Digitalisierung eines optischen Sensorsignals aus einem optischen Farbkanalsensor repräsentiert;
Bestimmen eines ersten Bildbereichs in mindestens einem Bild der Videobilder, wobei der erste Bildbereich einem Hautbereich der Person entspricht;
Bestimmen eines zweiten Bildbereichs in mindestens einem Bild der Videobilder, wobei der zweite Bildbereich einem anderen Bereich der Person als der erste Bildbereich entspricht;
Bestimmen eines Satzes von Farbkanalverteilungen für Pixel des ersten Bildbereichs;
Bereitstellen einer Hauttonangabe, wobei die Hauttonangabe einen Hautton der Person angibt;
Bereitstellen einer Referenzfarbkanalverteilungseigenschaft für den Hautton;
Bestimmen von Verstärkungen für die optischen Sensorsignale in Abhängigkeit von einer Luminanzeigenschaft, die für den zweiten Bildbereich bestimmt wird, und in Abhängigkeit von einer Differenz zwischen der Referenzfarbkanalverteilungseigenschaft und einer Eigenschaft des Satzes von Farbkanalverteilungen, wobei die bestimmten Verstärkungen so bestimmt werden, dass die Differenz reduziert wird;
Steuern von Verstärkungswerten, die auf die optischen Sensorsignale angewendet werden, so, dass sie mit den bestimmten Verstärkungen übereinstimmen; und
Schätzen einer Vitalzeicheneigenschaft für die Person aus den Videobildern.

15. Computerprogrammprodukt, das Computerprogrammcodemittel umfasst, die dazu ausgelegt sind, alle Schritte nach Anspruch 14 durchzuführen, wenn das Programm auf einem Computer ausgeführt wird.

## Revendications

1. Appareil pour estimer des signes vitaux d'une personne à partir d'images vidéo, l'appareil comprenant :
un récepteur (201) agencé pour recevoir des images vidéo comprenant une pluralité de canaux de couleur, chaque canal de couleur représentant une numérisation d'un signal de capteur optique provenant d'un capteur optique de canal de couleur ;
un premier détecteur (203) agencé pour déterminer une première zone d'image dans au moins une image des images vidéo, la première zone d'image correspondant à une zone de peau de la personne ;
un second détecteur (205) agencé pour déterminer une deuxième zone d'image dans au moins une image des images vidéo, la deuxième zone d'image correspondant à une zone de la personne différente de la première zone d'image ;
un dispositif de détermination (207) agencé pour déterminer un ensemble de distributions de canaux de couleur pour des pixels de la première zone d'image ;
un processeur (209) agencé pour fournir une indication de teint de peau, l'indication de teint de peau indiquant un teint de peau de la personne ;
un processeur de référence (211) agencé pour fournir une propriété de distribution de canaux de couleur de référence pour le teint de peau ;
un processeur de gain (213) agencé pour déterminer des gains pour les signaux de capteur optique en fonction d'une propriété de luminance déterminée pour la deuxième zone d'image et en fonction d'une différence entre la propriété de distribution de canaux de couleur de référence et une propriété de l'ensemble de distributions de canaux de couleur, les gains déterminés étant adaptés pour réduire la différence ;
un dispositif de commande de gain (215) agencé pour commander des valeurs de gain appliquées aux signaux de capteur optique pour correspondre aux gains déterminés ; et
un dispositif de détermination de signes vitaux (217) agencé pour estimer une propriété de signes vitaux pour la personne à partir des images vidéo.

2. Appareil selon la revendication 1 comprenant en outre :
un circuit de gain (103) agencé pour appliquer les valeurs de gain aux signaux de capteur optique provenant d'au moins deux capteurs optiques de canal de couleur ; et
un générateur d'images (105) couplé au circuit de gain (103) et agencé pour générer les images vidéo à partir des signaux de capteur optique reçus en provenance du circuit de gain (103).

3. Appareil selon l'une quelconque des revendications précédentes, la propriété de distribution de canaux de couleur de référence comprenant un ensemble de valeurs d'intensité de référence pour différents canaux de couleur, et la propriété de la distribution de canaux de couleur comprenant un ensemble de valeurs d'intensité déterminées pour l'ensemble de distributions de canaux de couleur.

4. Appareil selon la revendication 3, les valeurs d'intensité déterminées étant des valeurs de pixels de canal de couleur pour un percentile donné de l'ensemble de distributions de canaux de couleur.

5. Appareil selon la revendication 3 ou 4, le processeur de gain (213) étant agencé pour déterminer un gain pour un premier canal de couleur de la pluralité de canaux de couleur en fonction d'une différence entre une valeur d'intensité déterminée pour le premier canal de couleur et une valeur d'intensité de référence pour la valeur du premier canal de couleur.

6. Appareil selon l'une quelconque des revendications 3 à 5, le processeur de gain (213) étant agencé pour régler les gains de telle sorte qu'une relation entre des valeurs d'intensité déterminées pour les différents canaux de couleur corresponde à une relation entre des valeurs d'intensité de référence pour les différents canaux de couleur.

7. Appareil selon l'une quelconque des revendications 3 à 5, la propriété de luminance étant une luminance pour la deuxième zone d'image résultant de l'application des gains déterminés à la deuxième zone d'image, et le processeur de gain (213) étant agencé pour régler les gains sous réserve que la propriété de luminance soit indicative d'une luminance pour la deuxième zone d'image satisfaisant un critère.

8. Appareil selon l'une quelconque des revendications précédentes, le processeur de gain (213) étant agencé pour déterminer une composante de gain commune en fonction de la propriété de luminance et des composantes de gain relatives en fonction de la comparaison de la propriété de distribution de canaux de couleur de référence et d'une propriété de l'ensemble de distributions de canaux de couleur, la composante de gain commune étant un gain appliqué à tous les canaux de couleur de la pluralité de canaux de couleur et les composantes de gain relatives étant des composantes de gain appliquées à des canaux de couleur individuels de la pluralité de canaux de couleur.

9. Appareil selon l'une quelconque des revendications précédentes, le processeur de gain (213) étant agencé pour commander une propriété d'exposition d'au moins un capteur optique de canal de couleur en fonction d'au moins l'une de la propriété de luminance et de la comparaison de la propriété de distribution de canaux de couleur de référence et d'une propriété de l'ensemble de distributions de canaux de couleur.

10. Appareil selon l'une quelconque des revendications précédentes, le processeur de gain (213) étant agencé pour déterminer et mémoriser une première distribution de canaux de couleur pour une troisième zone d'image d'une image pour laquelle une zone de peau a été détectée ; et pour déterminer des gains pour les signaux de capteur optique pour une première image pour laquelle aucune zone de peau n'a été détectée en fonction de la première distribution de canaux de couleur et d'une seconde distribution de canaux de couleur déterminée pour une troisième zone d'image pour la première image.

11. Appareil selon la revendication 10, le processeur de gain (213) étant agencé pour régler le gain afin de réduire une différence entre une propriété de la première distribution de canaux de couleur et la seconde distribution de canaux de couleur.

12. Appareil selon la revendication 10 ou 11, la troisième zone d'image étant une zone d'image complète.

13. Appareil selon l'une quelconque des revendications précédentes, le processeur de gain (213) étant agencé pour déterminer des gains initiaux pour les signaux de capteur optique en fonction de la comparaison d'une valeur d'intensité d'image complète à une valeur d'intensité de référence prédéterminée ; la valeur d'intensité d'image complète étant une valeur d'intensité pour un percentile prédéterminé d'un canal de couleur de la pluralité de canaux de couleur.

14. Procédé, mis en œuvre par ordinateur d'estimation de signes vitaux d'une personne à partir d'images, le procédé comprenant
la réception d'images vidéo comprenant une pluralité de canaux de couleur, chaque canal de couleur représentant une numérisation d'un signal de capteur optique provenant d'un capteur optique de canal de couleur ;
la détermination d'une première zone d'image dans au moins une image des images vidéo, la première zone d'image correspondant à une zone de peau de la personne ;
la détermination d'une deuxième zone d'image dans au moins une image des images vidéo, la deuxième zone d'image correspondant à une zone de la personne différente de la première zone d'image ;
la détermination d'un ensemble de distributions de canaux de couleur pour des pixels de la première zone d'image ;
la fourniture d'une indication de teint de peau, l'indication de teint de peau indiquant un teint de peau de la personne ;
la fourniture d'une propriété de distribution de canaux de couleur de référence pour le teint de peau ;
la détermination de gains pour les signaux de capteur optique en fonction d'une propriété de luminance déterminée pour la deuxième zone d'image et en fonction d'une différence entre la propriété de distribution de canaux de couleur de référence et une propriété de l'ensemble de distributions de canaux de couleur, les gains déterminés étant déterminés de manière à réduire la différence ;
la commande des valeurs de gain appliquées aux signaux de capteur optique pour correspondre aux gains déterminés ; et
l'estimation d'une propriété de signes vitaux pour la personne à partir des images vidéo.

15. Produit de programme informatique comprenant des moyens de code de programme informatique adaptés pour réaliser toutes les étapes selon la revendication 14 lorsque ledit programme est exécuté sur un ordinateur.
